# EUROPÄISCHE PATENTANMELDUNG

(11) **EP 2 359 747 A1**
(43) Veröffentlichungstag der Anmeldung: **24.08.2011**
(21) Anmeldenummer: 10450195.2
(22) Anmeldetag: 17.12.2010
(51) Int. Cl.: A61B 5/00, A61B 5/103

(54) **Verfahren zur objektivierten Bestimmung und Messung von Schmerzen**

(30) Priorität: 12.02.2010 AT 2122010
(71) Anmelder: AIT Austrian Institute of Technology GmbH, 1220 Wien (AT)
(72) Erfinder: Oberleitner, Andreas, Dipl.-Ing., 2492 Zillingdorf (AT)
(74) Vertreter: Wildhack & Jellinek

(57) **Zusammenfassung**

Die Erfindung betrifft ein Verfahren zur objektivierten Bestimmung und Messung von Schmerzen und ist dadurch gekennzeichnet, dass ein von einer Person (A) auf eine Messvorrichtung (3, 4, 5) ausgeübter Druck (D) gemessen und als quantitatives Maß (S) für den Schmerz herangezogen wird, wobei der Druck (D) mit den von der Person (A) empfundenen Schmerzen korreliert wird. (Fig. 1)

## Beschreibung

Die Erfindung betrifft ein Verfahren zur objektivierten Bestimmung und Messung von Schmerzen.

Aus dem Stand der Technik sind verschiedene Vorrichtungen und Verfahren zur Schmerzmessung bzw. zur Erfassung des Schmerzempfindens bekannt. Aufgrund der Tatsache, dass das Schmerzempfinden subjektiv und individuell ist, hängt die Beurteilung in der Regel von der Schmerzäußerung des Patienten ab. Der Arzt oder Therapeut bei der Behandlung ist also von den subjektiven Schilderungen des Patienten abhängig, die allerdings starken Schwankungen unterworfen sind. Eine direkte Messung des Schmerzes ist nicht möglich.

Aus dem Stand der Technik sind verschiedene Methoden zur Objektivierung bzw. Quantifizierung von Schmerz bekannt, beispielsweise bewerten Patienten ihre Schmerzempfindung auf einer numerischen Skala von 1 bis 10, auf einer farblichvisuellen Analogskala mittels eines Schiebers, auf einer Smiley-Skala oder mittels Beschreibung durch Adjektive.

Auch existieren komplexe medizinische Methoden über die Messung der Aktionspotentiale von Nervenfasern, die Erfassung von Veränderungen der Mimik, der Messung von zerebralen Funktionen oder der Stoffwechselaktivitäten des Gehirns. Diese Parameter sind allerdings relativ unzuverlässig, hochkomplex, teuer und noch nicht ausreichend erprobt.

Zur Erfassung des Schmerzempfindens existieren sogenannte Algometer, mit welchen ein definierter Druck beispielsweise über ein Filament auf eine kleinflächige Stelle des Körpers aufgebracht wird. Durch die obigen subjektiven Rückmeldungen des Patienten können auf diese Weise Schmerzschwellen, d.h. insbesondere der Beginn der Empfindung in Proportion zum aufgebrachten Druck, registriert werden.

Aufgabe der vorliegenden Erfindung ist es somit, ein Verfahren zur objektiven Schmerzmessung zu schaffen, das verlässlich Auskünfte für die von einer Person empfundenen Schmerzen liefert und unabhängig von einer audiovisuellen Rückmeldung der Person ist.

Diese Aufgabe wird durch die kennzeichnenden Merkmale des Anspruches 1 gelöst. Hierbei ist vorgesehen, dass ein von einer Person auf eine Messvorrichtung ausgeübter Druck gemessen und als quantitatives Maß für den Schmerz herangezogen wird, wobei der Druck mit den von der Person empfundenen Schmerzen korreliert wird.

Es hat sich überraschenderweise herausgestellt, dass die Heranziehung des Druckes, der von einer Person auf einer Messvorrichtung ausgeübt wird, ein äußerst zuverlässiges und objektives Maß dafür ist, welche Schmerzen diese Person empfindet. Auf diese Weise braucht sich die Person weder verbal mitteilen, noch ist sie auf die visuelle Erfassung oder Widergabe von Sekundärzeichen angewiesen. Die Ausübung des Druckes auf eine Messvorrichtung erfolgt intuitiv und unbewusst, wobei die Druckkraft mit dem empfundenen Schmerz direkt proportional korreliert. Die Ermittlung des Schmerzes ist somit objektiv, zuverlässig und beeinträchtigt die Person nicht.

Insbesondere funktioniert dieses Verfahren auch bei Personen, die sich nicht artikulieren können und die Augen geschlossen haben, oder sich in einem komaartigen Zustand am Rande der Bewusstlosigkeit befinden.

Vorteilhafte Ausgestaltungen des Verfahrens werden durch die Merkmale der abhängigen Ansprüche beschrieben:
So ist gemäß einer vorteilhaften Weiterentwicklung des Verfahrens vorgesehen, dass der ausgeübte bzw. gemessene Druck und/oder das Maß erfasst und als von den von der Person empfundenen Schmerzen abhängig angesehen und ausgewertet werden, wobei insbesondere ein hoher ausgeübter Druck großen Schmerzen zugeordnet wird. Durch die Korrelation des ausgeübten Druckes mit den tatsächlichen Schmerzempfinden kann eine zuverlässige Beurteilung erreicht werden.

Das erfindungsgemäße Verfahren erlaubt es in vorteilhafter Weise, dass der ausgeübte bzw. gemessene Druck und/oder das Maß für den Schmerz kontinuierlich aufgezeichnet werden. Dies ist bei dem Verfahren im Stand der Technik nicht oder nur sehr eingeschränkt möglich. Durch die Vorsehung dieses Merkmals ist somit eine lückenlose kontinuierliche Aufzeichnung möglich.

Eine weitere vorteilhafte Ausgestaltung des Verfahrens sieht vor, dass der von der Hand bzw. Handfläche oder von Teilen der Hand, insbesondere den Fingern, der Person auf die Messvorrichtung ausgeübte Druck, insbesondere der Druck im Inneren der Faust, gemessen und als Maß für den Schmerz herangezogen wird. Die Verwendung einer solchen Messvorrichtung ist hygienisch, nicht invasiv und einfach zu bedienen.

Alternativ oder zusätzlich kann vorgesehen sein, dass der vom Kiefer und/oder den Zähnen der Person auf die Messvorrichtung im Zuge einer Beißbewegung ausgeübte Druck gemessen und als Maß für den Schmerz herangezogen wird. Auf diese Weise ist ebenfalls eine zuverlässige, intuitive und genaue Erfassung des Schmerzes möglich.

Vorteilhaft ist es auch, wenn zur Messung des ausgeübten Drucks eine, vorzugsweise zylindrische und/oder elastische, Messvorrichtung verwendet wird, die zumindest ein drucksensitives Element, beispielsweise ein kapazitiver oder piezoresitiver Sensor, aufweist, wobei die Messgröße des drucksensitiven Elements als Maß für den Schmerz herangezogen wird.

Gemäß einer weiteren vorteilhaften Verfahrensführung ist es möglich, dass vor der Bestimmung des Schmerzes eine Kalibrierungsmessung durchgeführt wird, bei der die Person subjektiv maximalen Druck auf die Messvorrichtung ausübt, wobei die anschließend gemessenen Werte des ausgeübten Drucks zu diesem subjektiven maximalen Druck in Relation gesetzt und bewertet werden und das Verhältnis, insbesondere der Quotient, zwischen dem Druck und dem vorab bestimmten subjektiv maximalen Druck als Maß für den Schmerz herangezogen wird.

Außerdem ist es möglich, dass vor der Bestimmung des Schmerzes eine Kalibrierungsmessung durchgeführt wird, wobei die Person die Messvorrichtung locker hält, ohne nach ihrem subjektiven Empfinden Druck auszuüben, und der dabei dennoch auf die Messvorrichtung ausgeübte Druck gemessen und als Haltedruck herangezogen wird, und der anschließend gemessene Druck abzüglich des vorab bestimmten Haltedrucks als Maß für den Schmerz herangezogen wird.

Durch diese Kalibrierungsmessungen können objektive Schmerzquantitäten festgestellt werden.

In weiterer Folge ist vorteilhaft, wenn vorgesehen ist, dass für eine Anzahl von Personen jeweils das Maß für den Schmerz und ein von der jeweiligen Person auf einer vorgegebenen numerischen Skala angegebener nach dem subjektiven Empfinden der Person festgelegter subjektiver Schmerzwert ermittelt werden, dass für die gemeinsam ermittelten Maße und Schmerzwerte eine, insbesondere lineare, Ausgleichsfunktion ermittelt wird, die einen Zusammenhang zwischen dem Maß für den Schmerz und dem subjektiven Schmerzwert angibt, und dass anschließend für eine Person das Maß für den Schmerz ermittelt und der Ausgleichsfunktion unterzogen wird und der so entstehende Funktionswert als weiteres Maß für den Schmerz herangezogen wird.

Eine weitere vorteilhafte Verfahrensführung ist dadurch gekennzeichnet, dass der Person vorab aufgetragen wird, einen von ihren Schmerzen abhängigen Druck auf die Messvorrichtung aufzubringen und der von der Messvorrichtung abgeleitete Druck in Hinblick auf eine Feststellung des Schmerzmaßes ausgewertet wird.

Die erfinderische Verfahrensführung sowie die dafür verwendeten Messeinrichtungen werden in den folgenden vorteilhaften Beispielen exemplarisch beschrieben und in den Figuren beispielhaft und nicht einschränkend gezeigt:
Fig. 1 zeigt eine erste Ausführungsform der Erfindung, die während einer zahnärztlichen Behandlung Anwendung findet.
Fig. 2 zeigt eine erste mögliche Ausführungsform einer Messvorrichtung zur Bestimmung der Handkraft.
Fig. 3 zeigt eine zweite mögliche Ausführungsform zur Bestimmung der Handkraft.
Fig. 4 zeigt eine Messvorrichtung zur Bestimmung des durch eine Beißbewegung ausgeübten Drucks.

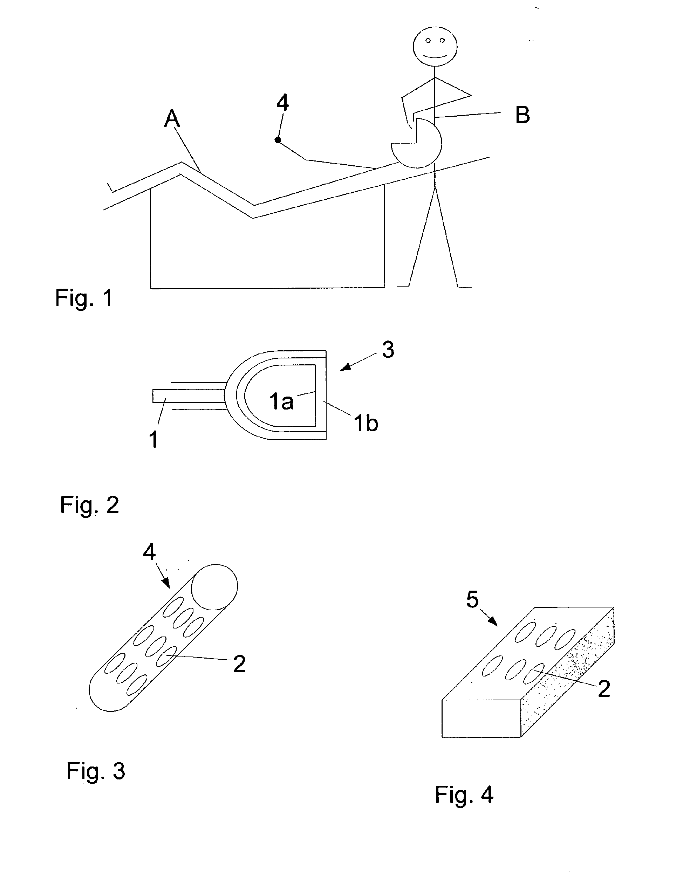

Ein typischer Einsatzbereich der Erfindung ist, wie in Fig. 1 dargestellt, eine Situation, in der für bestimmte Zwecke, beispielsweise während einer ärztlichen Behandlung oder zur Überwachung des allgemeinen Zustands einer Person A, ein objektives Schmerzmaß S für die Person A, das heißt ein objektives Maß dafür, welche Schmerzen eine Person empfindet, ermittelt werden soll. Dies kann beispielsweise während einer zahnärztlichen Behandlung vorgenommen werden, bei der zusätzlich das Problem besteht, dass der Patient während der Behandlung nicht sprechen kann und somit für ihn keine Mitteilungsmöglichkeit besteht.

Im vorliegenden Ausführungsbeispiel der Fig. 1 ist eine Person A dargestellt, die von einem Zahnarzt B behandelt wird. Die Person A hält eine, in Fig. 3 dargestellte Messvorrichtung 4 in der Hand. Vorteilhafterweise weist der Zahnarzt B die Person A vor Beginn der Behandlung darauf hin, dass es sich bei der Messvorrichtung 4 um eine Schmerzmesseinrichtung handelt, die die Handkraft bestimmt und abhängig von dem von der Person A aufgebrachten Druck das Schmerzmaß S für die Person A ermittelt. Dabei werden großen Drücken jeweils große Schmerzen zugeordnet. Somit besteht für die Person A während der Behandlung, bei der die Person A schlecht oder eingeschränkt kommunizieren kann, die Möglichkeit, dem Zahnarzt B mitzuteilen, ob und in welchem Ausmaß die Person A Schmerzen empfindet.

Während der Behandlung werden die Schmerzen kontinuierlich aufgezeichnet. Dabei kann der Zahnarzt B während der Behandlung laufend feststellen, ob die Behandlung für die Person A schmerzhaft ist und ob Schmerzen der Person A stärker bzw. schwächer werden, bzw. wann und ob bestimmte Grenzen überschritten werden.

Zur Bestimmung des Schmerzmaßes S können unterschiedliche Messvorrichtungen 3,4,5, wie in den Fig. 2, 3 und 4 dargestellt, herangezogen werden. Im Beispiel der Fig. 1 wird eine Messvorrichtung 4 herangezogen, die im Wesentlichen zylindrisch und elastisch aufgebaut ist. Die zylindrische Form weist den Vorteil auf, dass sie einfach in der Hand liegt, von der Hand zumindest teilweise umspannbar ist und gut von der Person gehalten werden kann. Die Elastizität hat den Vorteil, dass die Handgelenke bei auftretenden stärkeren Schmerzen nicht geschädigt werden. Am äußeren Mantel der Schmerzmessvorrichtung 4 sind drucksensitive Elemente 2, insbesondere FSR, kapazitive Sensoren, elektromechanische Drucksensoren oder Piezosensoren, angeordnet. Diese Sensoren sind vorteilhafterweise über den gesamten Umfang des Zylinders verteilt.

Eine weitere alternative Ausführungsform zur Bestimmung der Handkraft ist in Fig. 2 dargestellt. Diese Ausführungsform ist an sich bekannt und wird für Maximalkraftmessungen der Handkraft verwendet. Dabei umfasst die Messvorrichtung 3 einen starren Handgriff 1b und einen über einen Kolben 1 gelagerten, gegenüber dem Handgriff 1 b verschiebbaren zweiten Handgriff 1 b. Je nach Einstellung des Kolbens 1 wird der Handkraft der Person A ein mit der Auslenkung gegenüber der Ruhelage steigender Widerstand entgegengesetzt. Die Person A umgreift mit den Fingern die beiden Handgriffe 1a und 1b und versucht, durch Ballen der Faust die beiden Handgriffe 1a und 1b einander möglichst anzunähern. Über den Kolben 1 kann die Druckkraft abgenommen und einer weitern Verarbeitung zugeführt werden.

Eine weitere Ausführungsform der Erfindung zeigt gemäß Fig. 4 eine Messanordnung 5 zur Messung der Beißkraft einer Person A. Es ist im Rahmen einer Behandlung bzw. einer Überwachung des allgemeinen Zustands einer Person A durchaus erforderlich, die Schmerzen kontinuierlich aufzuzeichnen und zu messen. Eine Möglichkeit besteht darin, auf einer Zahnreihe einer Person A eine Messvorrichtung anzuordnen, aufzukleben oder aufzustecken. Ein typischer Schmerzreflex einer Person A ist das Zusammenbeißen der Zähne. Beim Zusammenbeißen werden die auf der Messvorrichtung 5 befindlichen drucksensitiven Elemente 2 zusammengedrückt, wodurch ein unterschiedlicher Spannungs- bzw. Widerstandswert der drucksensitiven Elemente 2 gemessen wird. Diese Werte können in einen Druckwert D umgerechnet werden, der als Maß S für die Schmerzen herangezogen wird.

Vorteilhafterweise kann vor Beginn der Behandlung der von der Person A subjektiv ausübbare maximale Druck M bestimmt werden. Anschließend werden sämtliche mit der Messvorrichtung 3, 4, 5 gemessenen Druckwerte D zu dem maximalen Druck M in Verhältnis gesetzt. Dabei wird insbesondere der Quotient zwischen dem gemessenen Druck D und dem subjektiv maximalen Druck M, der im Zuge der Kalibrierungsmessung bestimmt worden ist, als Maß S für den Schmerz herangezogen. Durch dieses Verfahren kann verhindert werden, dass kräftigeren Personen A grundsätzlich größere Schmerzen zugeordnet werden. Es ist somit eine genauere Schmerzbestimmung möglich.

Auch kann ein weiterer Kalibrierungsschritt vorab durchgeführt werden, bei dem die Person A die Messvorrichtung 3, 4, 5 nach subjektivem Empfinden locker in der Hand hält. Die Person A übt dabei keinen bewussten, schmerzkorrelierten Druck aus. Der dennoch ausgeübte Druck, im Folgenden Haltedruck H genannt, der mit der Messvorrichtung 3, 4, 5 ermittelt werden kann, resultiert einerseits aus der eigenen Gewichtskraft der Messvorrichtung 3, 4, 5 beim Halten in der Hand bzw. am Kiefer sowie aus Restspannungen der Hand bzw. des Kiefers der Person A, hauptsächlich hervorgerufen aufgrund der Fixierung der Messvorrichtung. Dieser Haltedruck H wird vom gemessenen Druck D abgezogen, um unterschiedliche Muskelrestspannungen unterschiedlicher Personen A aus der Messung auszufiltern.

Ein weiteres Kalibrierverfahren kann durchgeführt werden, indem für eine Anzahl von Personen A jeweils das Maß S für den Schmerz herangezogen wird. Die Person A wird gleichzeitig ersucht, einen nach subjektivem Empfinden festgelegten subjektiven Schmerzwert W anzugeben. Das jeweilige Maß S für den Schmerz wird mit dem jeweiligen von der Person A subjektiv festgelegten subjektiven Schmerzwert W koordiniert und gegebenenfalls in einer Tabelle abgespeichert. Für die gemeinsam ermittelten Maße S und Schmerzwerte W wird eine Ausgleichsfunktion F ermittelt, die einen Zusammenhang zwischen dem Maß S für den Schmerz und dem subjektiven Schmerzwert W angibt. Dabei kann vorab festgelegt werden, ob eine der zuvor genannten Kalibrierungen für sämtliche Personen A durchgeführt wird. Beispielsweise kann der Haltedruck bei allen Personen A abgezogen werden oder die gemessenen Schmerzwerte im Verhältnis zum subjektiv maximalen Druck S angegeben werden. Gegebenenfalls kann auch eine Kombination verwendet werden, wobei hierbei vom ausgeübten Druck D zunächst der Haltedruck H abgezogen wird und anschließend diese Druckdifferenz zum Maximalen Druck M abzüglich dem Haltedruck H der jeweiligen Person gesetzt wird.

Die Ausgleichsfunktion F wird dabei insbesondere linear angesetzt, um einen möglichst einfachen Zusammenhang zu erhalten. Gegebenenfalls können jedoch auch durchaus unterschiedliche Ausgleichsfunktionen, beispielsweise Polynomkurven oder Exponentialfunktionen herangezogen werden.

Nach Ermittlung dieser Ausgleichsfunktion wird auf die gleiche Weise, wie auch die genannten Messungen zur Ermittlung der Ausgleichsfunktion durchgeführt worden sind, das Maß S für den Schmerz der jeweiligen Person A ermittelt. Dieses Maß wird der Ausgleichsfunktion F unterzogen und der so entstehende Funktionswert wird als weiteres Maß S' für den Schmerz herangezogen.

In weiterer Folge ist auf Basis der erhaltenen Daten auch die Ermittlung des "Schmerzintegrals" über die Zeit bzw. der "Schmerzarbeit" möglich.

## Patentansprüche

1. Verfahren zur objektivierten Bestimmung und Messung von Schmerzen, **dadurch gekennzeichnet, dass** ein von einer Person (A) auf eine Messvorrichtung (3, 4, 5) ausgeübter Druck (D) gemessen und als quantitatives Maß (S) für den Schmerz herangezogen wird, wobei der Druck (D) mit den von der Person (A) empfundenen Schmerzen korreliert wird.

2. Verfahren nach Anspruch 1, **dadurch gekennzeichnet, dass** der ausgeübte bzw. gemessene Druck (D) und/oder das Maß (S) erfasst und als von den von der Person (A) empfundenen Schmerzen abhängig angesehen und ausgewertet werden, wobei insbesondere ein hoher ausgeübter Druck (D) großen Schmerzen, insbesondere direkt proportional, zugeordnet wird.

3. Verfahren nach Anspruch 1 oder 2, **dadurch gekennzeichnet, dass** der ausgeübte bzw. gemessene Druck (D) und/oder das Maß (S) für den Schmerz kontinuierlich aufgezeichnet werden.

4. Verfahren nach einem der vorangehenden Ansprüche, **dadurch gekennzeichnet, dass** der von der Hand bzw. Handfläche oder von Teilen der Hand, insbesondere den Fingern, der Person (A) auf die Messvorrichtung (3, 4, 5) ausgeübte Druck (D), insbesondere der Druck (D) im Inneren der Faust, gemessen und als Maß (S) für den Schmerz herangezogen wird.

5. Verfahren nach einem der vorangehenden Ansprüche, **dadurch gekennzeichnet, dass** der vom Kiefer und/oder den Zähnen der Person (A) auf die Messvorrichtung (3, 4, 5) im Zuge einer Beißbewegung ausgeübte Druck (D) gemessen und als Maß (S) für den Schmerz herangezogen wird.

6. Verfahren gemäß einem der vorangehenden Ansprüche, **dadurch gekennzeichnet, dass** zur Messung des ausgeübten Drucks (D) eine, vorzugsweise zylindrische und/oder elastische, Messvorrichtung (3, 4, 5) verwendet wird, die zumindest ein drucksensitives Element (2), beispielsweise einen kapazitiven oder piezoresitiven Sensor, einen FSR- oder ein Luftdrucksensor, aufweist, wobei die Messgröße des drucksensitiven Elements als Maß (S) für den Schmerz herangezogen wird.

7. Verfahren gemäß einem der vorangehenden Ansprüche, **dadurch gekennzeichnet, dass** vor der Bestimmung des Schmerzes eine Kalibrierungsmessung durchgeführt wird, bei der die Person (A) subjektiv maximalen Druck (M) auf die Messvorrichtung (3, 4, 5) ausübt, wobei die anschließend gemessenen Werte des ausgeübten Drucks (D) zu diesem subjektiven maximalen Druck (M) in Relation gesetzt und bewertet werden und das Verhältnis, insbesondere der Quotient, zwischen dem Druck (D) und dem vorab bestimmten subjektiv maximalen Druck (M) als Maß (S) für den Schmerz herangezogen wird.

8. Verfahren gemäß einem der Ansprüche 1 bis 6, **dadurch gekennzeichnet, dass** vor der Bestimmung des Schmerzes eine Kalibrierungsmessung durchgeführt wird, wobei die Person (A) die Messvorrichtung (3, 4, 5) locker hält, ohne nach ihrem subjektiven Empfinden Druck auszuüben, und der dabei dennoch auf die Messvorrichtung (3, 4, 5) ausgeübte Druck gemessen und als Haltedruck (H) herangezogen wird, und der anschließend gemessene Druck (D) abzüglich des vorab bestimmten Haltedrucks (H) als Maß (S) für den Schmerz herangezogen wird.

9. Verfahren gemäß einem der vorangehenden Ansprüche, **dadurch gekennzeichnet, dass** für eine Anzahl von Personen (A) jeweils das Maß (S) für den Schmerz, insbesondere nach einem der vorangehenden Ansprüche, und ein von der Person (A) auf einer vorgegebenen numerischen Skala angegebener nach dem subjektiven Empfinden der jeweiligen Person (A) festgelegter subjektiver Schmerzwert (W) ermittelt werden, dass für die gemeinsam ermittelten Maße (S) und Schmerzwerte (W) eine, insbesondere lineare, Ausgleichsfunktion (F) ermittelt wird, die einen Zusammenhang zwischen dem Maß (S) für den Schmerz und dem subjektiven Schmerzwert (W) angibt, und
dass anschließend für eine Person (A) gemäß einem der vorangehenden Ansprüche das Maß (S) für den Schmerz ermittelt und der Ausgleichsfunktion (F) unterzogen wird und der so entstehende Funktionswert als weiteres Maß (S') für den Schmerz herangezogen wird.

10. Verfahren nach einem der vorangehenden Ansprüche, **dadurch gekennzeichnet, dass** der Person (A) vorab aufgetragen wird, einen von ihren Schmerzen abhängigen Druck auf die Messvorrichtung (3, 4, 5) aufzubringen und der von der Messvorrichtung (3, 4, 5) abgeleitete Druck in Hinblick auf eine Feststellung des Schmerzmaßes ausgewertet wird.
